# EUROPEAN PATENT SPECIFICATION

(11) **EP 0 733 608 B1**
(45) Date of publication and mention of the grant of the patent: **05.01.2000**
(21) Application number: 96104488.0
(22) Date of filing: 21.03.1996
(51) Int. Cl.: C07C 6/12, C07C 15/073, C07C 2/66

(54) **Transalkylation of polyalkylaromatic hydrocarbons**
Transalkylierung polyalkylaromatischer Kohlenwasserstoffe
Transalkylation d'hydrocarbures polyalkylaromatiques

(30) Priority: 21.03.1995 US 407222
(43) Date of publication of application: 25.09.1996
(73) Proprietor: FINA TECHNOLOGY, INC., Plano, Texas 75024 (US)
(72) Inventor: Kuchenmeister, Mark, Friendswood, Texas 77546 (US); Butler, James, Houston, Texas 77062 (US)
(74) Representative: Leyder, Francis

(56) References cited:
- US-A- 4 922 053

## Description

The present invention relates to a process for transalkylating polyalkylbenzenes such as diethylbenzenes produced during the alkylation of an aromatic hydrocarbon with an olefin using a catalyst with improved transalkylation activity.

### BACKGROUND OF THE INVENTION

Ethylbenzene is used primarily for the production of styrene monomer obtained through dehydrogenation. Currently, much of the ethylbenzene produced is obtained by alkylation of benzene with ethylene under a variety of alkylation conditions. One type of alkylation process involves vapor phase reactions in which benzene is alkylated with an olefin such as ethylene under high temperatures and pressures over a zeolite catalyst in a multiple bed reactor.

A significant problem in the production of ethylbenzene by alkylation of benzene with ethylene under high temperatures and pressures is the production of undesired byproducts, including polyalkylbenzenes such as polyethylbenzenes (PEB's). Although the production of PEB's may be limited to some extent by supplying a stoichiometric excess of benzene to the reactor, significant quantities of these materials are nevertheless generated during the alkylation process.

In conventional vapor phase alkylation of benzene with ethylene, the reactor effluent is subjected to successive fractionations to separate benzene, ethylbenzene, PEB's and heavy residues into separate streams. The PEB stream may be directed to a separate transalkylation reactor or recycled to the alkylation reactor.

In the case where the PEB stream is recycled to the alkylation reactor, the recycled PEB stream can constitute up to ten percent of the total feed to the alkylation reactor. Consequently, if the transalkylation activity of the catalyst is not sufficient to control the concentration of PEB's in the alkylation reactor effluent, the volume of recycled PEB's will increase and eventually become too large to manage within process capacity. Therefore, it is important that the catalyst used in the alkylation of benzene with ethylene in a process with PEB recycle to the alkylation reactor have sufficient transalkylation activity to convert recycled polyalkylbenzenes at a rate sufficient to maintain the volume of the recycled polyalkylbenzenes stream at a level within process capacity. For example, U.S. Patent n° 4,922,053 discloses a process for producing ethylbenzene from the catalytic alkylation of benzene in a multibed reactor wherein a portion of the normal overhead polyethylbenzene recycle stream is diverted into a lower section of the reactor to increase conversion and lower xylene by-product production.

In connection with the foregoing, it has been discovered that the use of an aluminosilicate alkylation catalyst having a crystal size within a specified range results in increased transalkylation activity, allowing for greater control of the volume of recycled alkylbenzenes in the process.

### SUMMARY OF THE INVENTION

The present invention provides a method for transalkylating polyalkylbenzenes that is particularly useful in an alkylation process in which benzene is reacted with ethylene to produce ethylbenzene under reaction conditions. The use of process of the present invention provides greater control of the amount of polyalkylbenzenes recycled in such a process, resulting in increased capability to maintain an acceptable level of recycled polyalkylbenzenes. The process comprises supplying a feedstock containing benzene and recycled polyalkylbenzenes into a reaction zone with an alkylating agent in a molar ratio of benzene to alkylating agent of from about 2:1 to about 20:1 and into contact with an aluminosilicate alkylation catalyst having an average crystal size of less than about 0.50 µm (0.50 microns). In one embodiment of the present invention, about 90% of the crystals have a particle size of less than 0.70 µm. The catalyst is further characterized by an Si/Al atomic ratio in the range from between 50 and 500, a maximum pore size in the range from about 1000 to 1800 A. The catalyst has a sodium content of less than about 50 ppm and the reaction is carried out under conversion conditions including a temperature of from about 250°C. to about 500°C. and a pressure of from about 1.379 10⁶ Pa (200 psi) to about 3.448 10⁶ Pa (500 psi). An effluent stream containing ethylbenzene is recovered from the reaction zone.

### BRIEF DESCRIPTION OF THE DRAWINGS

FIGURE 1 is a schematic illustration of process of the present invention;
FIGURE 2 is a graphical illustration of the size distribution of the crystal size of the catalyst used in the practice of the present invention; and
FIGURE 3 is a graphical illustration of the distribution of pore sizes of the catalyst used in the practice of the present invention.

### DETAILED DESCRIPTION

The process of the present invention can be carried out using a variety of process equipment, including a reactor vessel which defines a reaction zone containing catalyst material. Either single or multiple catalyst beds can be employed in the reaction zone. The ethylene and benzene reactants may be mixed and preheated prior to introduction to the reaction zone which may consist of one or more catalyst beds where the reactants contact the catalyst under reaction conditions. The reaction products are withdrawn from the reaction zone after a controlled residence time and the reaction products are collected and separated by conventional techniques. Excess benzene along with polyalkylbenzenes are typically recycled to the reaction zone.

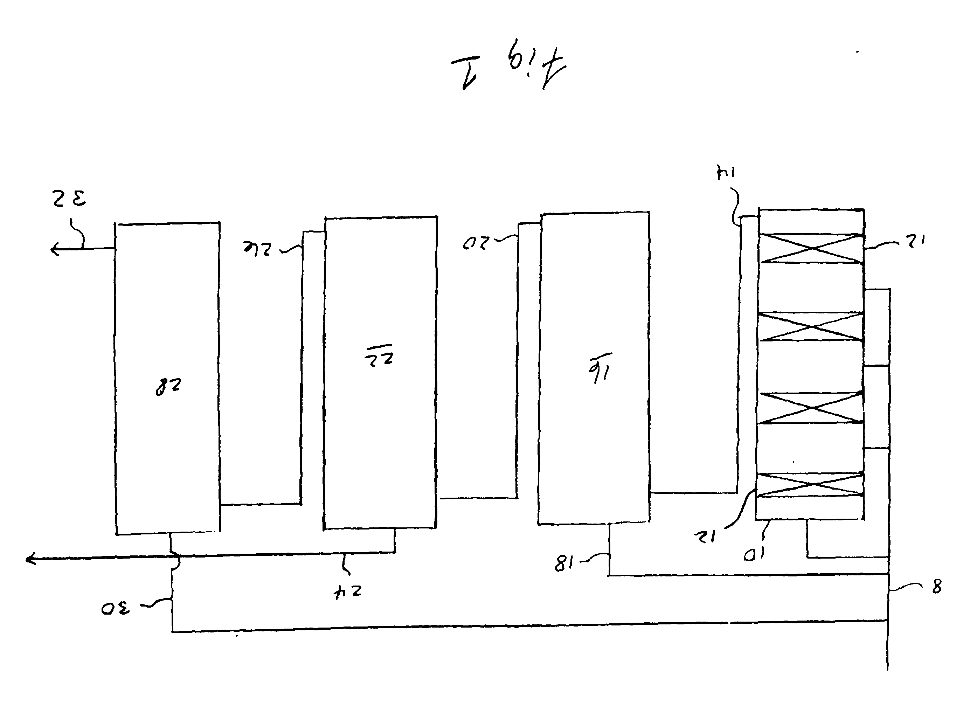

Turning now to FIGURE 1, one embodiment of the process of the present invention is schematically illustrated. A feed stream 8 containing benzene and an alkylating agent such as ethylene is injected into a multiple bed alkylation reactor 10 where the reactants are brought into contact with a catalyst in catalyst zones 12. Since the alkylation reaction is highly exothermic, the reactants may be injected at multiple locations in the reactor for process control purposes. An effluent stream 14 from the reactor 10 is directed to one or more benzene fractionating columns 16 where benzene is separated from the reactor effluent. The overhead stream 18 from the benzene fractionating column is recycled to the feed stream 8 to the alkylation reactor 10. The bottom stream 20 from the benzene fractionating column 16 is directed to an ethylbenzene fractionating column 22 where an ethylbenzene product stream 24 is separated and recovered. The bottom stream 26 from the ethylbenzene fractionating column 22 is directed to a third fractionating 28 column where an overhead stream 30, containing PEB's is separated and recycled to the alkylation reactor 10. Higher boiling point residual materials are recovered as the bottom stream 32 from the third fractionating column 28. In addition to PEB's, the overhead stream 30 may contain varying amounts of lower boiling point compounds such as xylenes, styrene, cumene, and propyl benzene.

The catalyst used in the process of the present invention is selective to the production of ethylbenzene in a temperature range of from about 250°c. to about 500°C. and a pressure range of from about 1.379 10⁶ Pa (200 psi) to about 3.448 10⁶ Pa (500 psi). The catalyst is an aluminosilicate powder with a crystalline structure that is primarily monoclinic aluminosilicate but which may contain up to about 40% orthorombic crystalline structure. In one embodiment of the present invention, the catalyst contains about 20% by weight of an alumina binder. Importantly, the catalyst has sufficient transalkylation activity to convert an amount of the recycled polyalkylbenzenes sufficient to maintain the volume of the recycled polyalkylbenzene stream at a level within conventional process capabilities.

The Si/Al atomic ratio of the catalyst is preferably in the range of from about 50 to about 500, more preferably in the range of from about 50 to 150 and even more preferably in the range of from about 70 to about 130. As used herein the Si/Al ratio refers to the atomic ratio of silicon to aluminum in the catalyst to the exclusion of the binder.

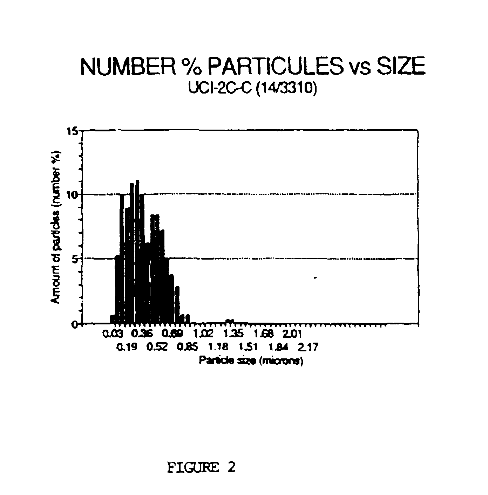

The improvement in transalkylation control of the process of the present invention is attributed to the use of an aluminosilicate catalyst having a crystal size less than about 0.50 µm (0.50 microns). In addition to the small crystal size of the catalyst, the distribution of particle sizes of the crystals is also narrow. As illustrated in FIGURE 2, the average crystal size of the catalyst is less than about 0.50 µm (0.50 microns), with approximately 90% of the crystals having a particle size of less than 0.70 µm. Catalysts with average crystal sizes exceeding the specified maximum average particle size of about 0.50 µm have proven to have lower transalkylation activity and therefore afford less control of the volume of polyalkylbenzenes recycled in the process.

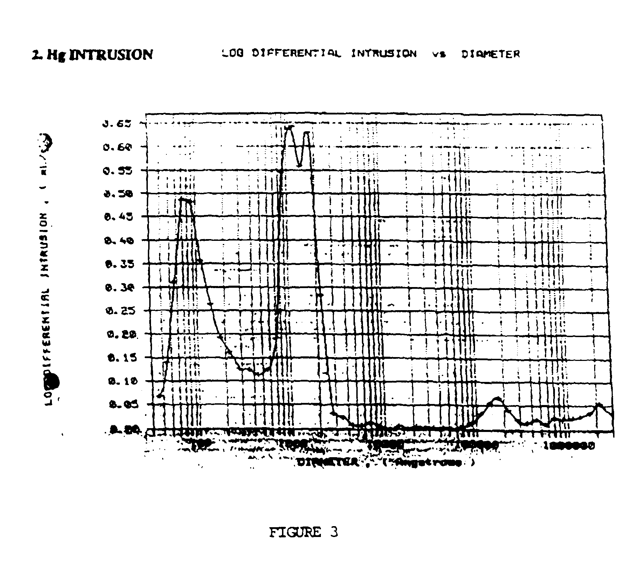

The pore size of the catalyst is also noteworthy, ranging from about 1000Å (1000 angstroms) to about 1800Å. FIGURE 3 illustrates the pore size distribution by volume for a catalyst used in the practice of the present invention. Additionally, the sodium content of the catalyst should be maintained at a level less than about 50 ppm in order to facilitate the desired levels of transalkylation.

In the process of the present invention, an excess of benzene to ethylene is normally maintained in the feed to the reactor, typically in the range of from about 2:1 to 20:1 moles benzene to ethylene, preferably between about 3:1 to 16:1. Weight hourly space velocities are preferably in the range from about 20 to 150. Although reaction conditions employing temperatures from about 250°C. to about 500°C. may be utilized, more preferably the reaction is conducted within a range of from about 300°C. to about 475°C.

The process of the present invention may be further illustrated by the following examples which are not to be construed as limiting the scope of the invention as hereinafter claimed.

### EXAMPLE 1

Ten milliliters of an aluminosilicate catalyst having a particle size distribution of between 20 and 40 mesh is introduced into a laboratory scale reactor. The catalyst has a crystallinity in the range of 76 to 93%, 64% monoclinicity, an average crystal size of 0.41 µm, a pore volume maxima at 1763 Å and an Si/Al atomic ratio of 114. The catalyst is heated under nitrogen flow to 150°C overnight at ambient pressure to dry the catalyst. The temperature is increased to 200°C and benzene with 10% mixed polyethylbenzenes is introduced to the reactor at a rate of 11.6 ml./min. The pressure is increased to 300 psig and the temperature raised to 400°C. After the temperature across the reactor bed has stabilized, ethylene is introduced at a rate of 1 mole per 10 moles of benzene feed. The test is run for 15 days without interruption. During the test, diethylbenzene (DEB's) concentrations in the feed and effluent from the reactor are measured and the percentage of diethylbenzenes converted, based on the DEB concentration in the feed, is calculated. The ethylene feed is shut off periodically to determine the intrinsic diethylbenzene conversion rate. The results are set forth in Table 1 below:

**TABLE 1**

| Test Duration (days) | % DEB's Converted | % DEB's Converted Without Ethylene Feed |
|---|---|---|
| 1.0 | 11.0 | 21.8 |
| 1.6 | 8.2 | - |
| 2.0 | 9.2 | 21.2 |
| 2.7 | 9.0 | - |
| 3.7 | 7.6 | - |
| 4.6 | 8.7 | 19.2 |
| 5.0 | 7.7 | - |
| 5.6 | 7.4 | - |
| 6.0 | 6.6 | - |
| 6.6 | 7.4 | - |
| 7.0 | 6.8 | - |
| 7.6 | 4.7 | 18.3 |
| 8.6 | 5.4 | - |
| 9.0 | 4.5 | - |
| 9.7 | 5.6 | - |
| 10.8 | 5.0 | |
| 11.6 | 5.0 | 15.4 |
| 12.0 | 3.8 | - |
| 12.6 | 3.7 | - |
| 12.9 | 1.1 | - |
| 14.0 | 3.6 | 16.5 |
| 14.6 | 2.8 | 16.2 |

The foregoing example illustrates the substantial transalkylation activity afforded by the use of an aluminosilicate catalyst having a crystal size in accord with the process of the present invention. The following Comparative Example will further illustrate the advantages of the process of the present invention.

### COMPARATIVE EXAMPLE 1

Ten milliliters of an aluminosilicate catalyst having a particle size distribution of between 20 and 40 mesh is introduced into a laboratory scale reactor. The catalyst has a crystallinity in the range of 77 to 88%, 70% monoclinicity, an average crystal size of 0.70 µm, a pore volume maxima at 3500 Å and an Si/Al atomic ratio of 91. The catalyst is heated under nitrogen flow to 200°C overnight at ambient pressure to dry the catalyst. The nitrogen flow is discontinued and benzene with 10% mixed polyethylbenzenes is introduced to the reactor at a rate of 11.6 ml./min. The pressure is increased to 300 psig and the temperature allowed to equilibrate at 400°C before introducing ethylene at a rate of 1 mole per 10 moles of benzene feed. The test is run for 8 days without interruption. During the test, diethylbenzene (DEB's) concentrations in the feed and effluent from the reactor are measured and the percentage of diethylbenzenes converted, based on the DEB concentration in the feed, is calculated. The results are set forth in Table 2 below:

**TABLE 2**

| Test Duration (days) | % DEB's Converted |
|---|---|
| 1.0 | 4.6 |
| 1.6 | 7.9 |
| 2.0 | 4.4 |
| 2.6 | 6.0 |
| 2.9 | 5.8 |
| 3.6 | 6.6 |
| 3.9 | 5.9 |
| 4.6 | 6.1 |
| 4.9 | 5.2 |
| 5.6 | 5.0 |
| 6.0 | 5.1 |
| 6.7 | 4.6 |
| 7.7 | 2.0 |

As can readily be observed by a comparison of the foregoing Examples, it is apparent that the use of an aluminosilicate catalyst having an average crystal size of less than about 0.50 µm provides increased transalkylation activity.

While the invention has been described in connection with the foregoing examples, it will be appreciated by the those skilled in the art that the invention is subject to variations and modifications which fall within the scope of the appended claims and which are intended to be covered thereby.

## Claims

1. A transalkylation process comprising :
transalkylating polyalkylbenzenes produced during the alkylation of an aromatic hydrocarbon with an alkylation agent in an alkylation zone with an aluminosilicate catalyst having an Si/Al atomic ratio in the range of between 50 and 500, an average crystal size of less than 0.50 µm under conversion conditions, with 90% of the crystals having a particle size less than 0.70 µm, and a maximum pore size of from 1000 to 1800 Å; and recovering ethylbenzene from the reaction zone.

2. The process of claim 1 wherein the conversion conditions include a temperature of from 250°C to 500°C and a pressure of from 1.379 10⁶ Pa (200 psi) to 3.448 10⁶ Pa (500 psi).

3. The process of claim 1 wherein the aromatic hydrocarbon is benzene and the alkylation agent is ethylene and wherein benzene is alkylated with ethylene to produce ethylbenzene.

4. The process of claim 3 wherein the molar ratio of benzene to ethylene in the feed to the reaction zone is from 2:1 to 20:1.

5. The process of claim 1 wherein the catalyst contains less than about 50 ppm sodium.

## Patentansprüche

1. Transalkylierungsverfahren, umfassend:
Transalkylieren von Polyalkylbenzolen, hergestellt während der Alkylierung eines aromatischen Kohlenwasserstoffes mit einem Alkylierungsagenz in einer Alkylierungszone, mit einem Aluminiumsilikatkatalysator mit einem Si/Al Atomverhältnis im Bereich zwischen 50 und 500, einer Durchschnittskristallgröße von weniger als 0,50 µm unter Umwandlungsbedingungen, wobei 90% der Kristalle eine Teilchengröße von weniger als 0,70 µm haben, und einer maximalen Porengröße von 1000 bis 1800 A, und Gewinnen von Ethylbenzol aus der Reaktionszone.

2. Verfahren nach Anspruch 1, wobei die Umwandlungsbedingungen eine Temperatur von 250°C bis 500°C und einen Druck von 1,379 x 10⁶ Pa (200 psi) bis 3,448 x 10⁶ Pa (500 psi) einschließen.

3. Verfahren nach Anspruch 1, wobei der aromatische Kohlenwasserstoff Benzol ist, und das Alkylierungsagenz Ethylen ist, und wobei Benzol mit Ethylen unter Herstellen von Ethylbenol alkyliert wird.

4. Verfahren nach Anspruch 3, wobei das Molverhältnis von Benzol zu Ethylen in der Zufuhr zu der Reaktionszone 2:1 bis 20:1 ist.

5. Verfahren nach Anspruch 1, wobei der Katalysator weniger als etwa 50 ppm Natrium enthält.

## Revendications

1. Un procédé de transalkylation comprenant:
transalkyler des polyalkylbenzènes produits pendant l'alkylation d'un hydrocarbure aromatique avec un agent d'alkylation dans une zone d'alkylation avec un catalyseur d'aluminosilicate ayant un rapport atomique Si/Al se situant entre 50 et 500, une dimension moyenne des cristaux de moins de 0,50 µm sous des conditions de conversion, 90% des cristaux ayant une dimension de particules de moins de 0,70 µm, et une dimension des pores maximum entre 1000 à 1800 Å, et récupérer l'éthylbenzène de la zone de réaction.

2. Le procédé de la revendication 1 dans lequel les conditions de conversion comportent une température de 250°C à 500°C et une pression de 1,379 10⁶ Pa (200 psi) à 3,448 10⁶ Pa (500 psi).

3. Le procédé de la revendication 1 dans lequel l'hydrocarbure aromatique est le benzène et l'agent d'alkylation est l'éthylène et dans lequel le benzène est alkylé avec l'éthylène pour produire de l'éthylbenzène.

4. Le procédé de la revendication 3 dans lequel le rapport molaire de benzène à éthylène dans le courant d'alimentation vers la zone de réaction est de 2:1 à 20:1.

5. Le procédé de la revendication 1 dans lequel le catalyseur contient moins de environ 50 ppm de sodium.
